# EUROPÄISCHE PATENTSCHRIFT

(11) **EP 0 702 955 B1**
(45) Veröffentlichungstag und Bekanntmachung des Hinweises auf die Patenterteilung: **11.04.2001**
(21) Anmeldenummer: 95250218.5
(22) Anmeldetag: 08.09.1995
(51) Int. Cl.: A61K 35/54

(54) **Verwendung von BK-RiV-Präparaten als Arzneimittel zur Therapie von AIDS**
Use of BK-RiV preparations as a drug for AIDS therapy
Utilisation des préparations à base de BK-RiV comme médicament pour la thérapie du SIDA

(30) Priorität: 20.09.1994 DE 4435352
(43) Veröffentlichungstag der Anmeldung: 27.03.1996
(73) Patentinhaber: Solisch, Peter, dr.med.habil., 17498 Riemserort (DE); Bergmann, Helmut, Dr.med.vet.sc., 17498 Riemserort (DE)
(72) Erfinder: Solisch, Peter, dr.med.habil., 17498 Riemserort (DE); Bergmann, Helmut, Dr.med.vet.sc., 17498 Riemserort (DE)
(74) Vertreter: Kolberg, Peter, Dr.

(56) Entgegenhaltungen:
- DD-A- 228 739
- DD-A- 268 159
- DD-A- 283 330

## Beschreibung

Die Erfindung betrifft die Verwendung von BK-RiV-Präparaten als Arzneimittel zur Therapie von AIDS. Die betreffenden Arzneimittel und ihre planmäßige Anwendung soll bei AIDS jedweder Ätiologie, aber insbesondere bei durch HIV ausgelösten Formen hochwirksam und in der Regel ohne Nebenwirkungen zur Anwendung gelangen und nicht nur - wie allgemein bekannt - zur Aufhebung der Symptome für einen gewissen Zeitraum führen, wie bei bekannten Arzneimitteln, die zur Behandlung von AIDS-Patienten eingesetzt werden, sondern könnte - aus theoretischer Sicht - nach jahrelanger Behandlung auch die Heilung bewirken.

Dem Stand der Technik entspricht das bisher weltweit einzige zugelassene Präparat AZT, das durch Eingriff in den Zellkernstoffwechsel von infizierten, aber auch nicht infizierten Zellen die Virusvermehrung behindert oder zeitweilig aufhebt und die Lebenserwartung für gewisse Zeiträume, die meist in Monaten bemessen sind, verbessern kann, aber bei einem Teil der Patienten erhebliche Nebenwirkungen verursacht (Übelkeit, Kopfschmerzen, Absinken der roten und weißen Blutzellen u.a.), was z.T. zum Abbruch der Behandlung oder/und zu lebenserhaltenden Maßnahmen wie Bluttransfusionen zwingt. Eine Heilung mit AZT wird nicht erwartet. Bisher ist ein spezifisches Schädigungsmuster - durch Lentiviren bedingt - nicht bekannt bzw. es wird nicht zum Prinzip einer neuartigen Bekämpfung dieses Krankheitsphänomens erhoben. Die Infektion durch ein Mitglied der Virusgattung Lentivirinae
- z.B. HIV - führt immer zum Tode des betreffenden Individuums. Wesentliche Teile der Pathogenese dieses schicksalhaften Verlaufes sind Allgemeingut der medizinischen Wissenschaft, die Berücksichtigung des von uns erkannten pathogenetischen Details fehlt.

Es wurde bereits vorgeschlagen, DD-PS 227 327, DD-PS 228 739, DD-PS 268 159, DD-PS 283 330, DE-OS 42 38 766 und DE-GbM G 92 18 127.9, RiV-Präparate (Reaktionsmuster in Vertebratenzellen) in Form eines Biokomplexes herzustellen und bei der Bekämpfung von Tumorerkrankungen und bei bis dahin bekannten "klassischen" Virusinfektionen, die eine grundsätzlich andere Pathogenese aufweisen als die HIV-Infektionen, einzusetzen. Ein Einsatz bei mit Sicherheit tödlich verlaufenden Lentivirusinfektionen war nicht vorgesehen.

Die Erfindung bezweckt, die Nachteile, die bei der AIDS-Behandlung mit den bekannten Arzneimitteln auftreten, zu vermeiden.

Der Erfindung liegt die Aufgabe zugrunde, die Krankheit AIDS durch die Anwendung eines bisher vor allem zur Tumortherapie vorgesehenen Arzneimittels - ohne Nebenwirkungen als physiologische Substitutionstherapie - die zum Tode führende Pathogenese der HIV-Infektion im betroffenen Patienten aufzuheben, die Anwesenheit des HIV durch Langzeittherapie zunehmend einzuschränken und schließlich auszuschließen, die unspezifische und spezifische Abwehrreaktion des Organismus wieder herzustellen (letzteres gilt auch für AIDS anderer als HIV-Infektion bedingter Ätiologie) und damit weitgehende Symptomfreiheit oder sogar Heilung anzustreben.

Es wurde überraschend gefunden, dass mit großer Wahrscheinlichkeit das Reaktionsmuster in Vertebratenzellen (RiV) bei Lentivirusinfektionen selektiv ausgeschaltet und damit der schicksalhafte letale Verlauf dieser Infektionen begründet wird. Es wurde gefunden, dass das isolierte RiV -in Form des Präparates BK-RiV, das nach den bereits vorgeschlagenen Herstellungsverfahren, die in den Schutzrechten DD-PS 227 327, DD-PS 228 739, DD-PS 268 159, DD-PS 283 330, DE-OS 42 38 766 und in dem DE-GbM G 92 18 127.9 offenbart sind, produziert werden, bei AIDS angewendet werden können. Es wurde weiterhin gefunden, dass die therapeutische Anwendung von BK-RiV bei an AIDS Erkrankten ohne jede Nebenwirkungen war, dass BK-RiV in der Lage war, im peripheren Blut die roten Zellen leicht in der Norm zu erhöhen, die CD-4-Zellen zu verdoppeln (z. B. von 150 auf über 300/µl), die Monozyten an die obere Grenze der Norm zu führen, die CD-8- und NK-Zellen innerhalb der Norm zu verdreifachen, das Allgemeinbefinden von "schwerkrank" auf subjektiv "arbeitsfähig" zu verbessern und den Prozess über 100 Tage (bisher bekannt) stabil und aufbauend zu erhalten. Die multiplen subjektiven Beschwerden des Patienten verschwanden unter der Therapie vollkommen, die psychologische Situation des Patienten normalisierte sich.

Der BK wird aus geeignetem Material für Zellkulturen, wie Embryonen, Feten ausgenommen menschliche Embryones und Feten oder Wirbeltieren bzw. aus deren Gewebe, Organen oder Tumoren, aus denen begrenzt oder unbegrenzt wachsende Zellkulturen hergestellt werden können, gewonnen. Diese Zellkulturen werden durch Kultivierung oder gezielten Einsatz biologischer, wie protahierter Kultivierung, chemischer, wie Glucoseunterversorgung, oder physikalischer Mittel, wie die Wahl einer Temperatur außerhalb des physiologischen Bereichs, zur Bildung des partikulären Biokomplexes (BK) stimuliert. Aus der Kulturflüssigkeit werden die Zellsedimente unter weitgehender Zellerhaltung gewonnen. Danach wird eine Zelldestruktion unter Erhaltung des BK durchgeführt.
Der BK wird durch fraktionierte Zentrifugation, Ultrazentrifugation und durch Extraktion mit vorzugsweise Chloroform oder Ether oder durch fraktionierte Fällungsverfahren und/oder andere Stofftrennungsverfahren abgetrennt. Dieser Schritt wird durch Sichtkontrolle unter dem Elektronenmikroskop im Negativkontrastverfahren begleitet.

Das Endziel des Herstellungsverfahrens von BK ist, eine wasserklare, gepufferte Lösung (Gesamt-Eiweißgehalt < 0,875 mg/ml) zu erhalten, deren elektronenmikroskopisches Bild im Negativkontrastverfahren ausschließlich aus sphärischen oder nur geringgradig pleomorphen im Mittel etwa 50 nm großen "vollen" und "leeren" Partikeln sowie weiteren im Mittel etwa 9 nm großen sphärischen oder pleomorphen in der Regel "leeren" Partikeln repräsentiert wird.

Das Herstellungsverfahren besteht aus folgenden Schritten:
- Suche, Prüfung und Auswahl der Ausgangsspender für Zellkulturen, wie Embryonen, Feten, ausgenommen menschliche Embryonen und Feten oder Tiere bzw. deren Gewebe, Organe oder Tumore;
- Herstellung von begrenzt oder unbegrenzt wachsenden Zellkulturen;
- Stimulierung der Zellkulturen durch die Art und Weise der Kultivierung oder gezielter Einsatz biologischer, chemischer oder physikalischer Mittel;
- Gewinnung der Zellsedimente je nach Kultivierungsbedingungen unter weitgehender Zellerhaltung ohne Zusatz das Endprodukt belastender Stoffe;
- Zelldestruktion unter alleiniger Erhaltung von BK;
- Durchführung fraktionierter Zentrifugation, Ultrazentrifugation und Extraktion unter Sichtkontrolle mit dem Elektronenmikroskop im Negativkontrastverfahren;
- oder alternativ Durchführung von fraktionierten Fällungsverfahren und/oder anderer Stofftrennungsverfahren;
- Prüfung auf Identität und Unschädlichkeit.

Die Kontinuität und extreme Dimension massenhafter Zellkultivierung setzt eine ökonomische Bereitstellung der Ausgangsspender voraus, die bekannter, veterinärmedizinisch kontrollierter Herkunft sein müssen. Bevorzugt kommen in Betracht:
- Nieren neugeborener Kälber,
- Nieren von Schweinefeten,
- Embryonen von Enten oder Hühnern, wobei die Verwendung von menschlichen Embryonen ausdrücklich ausgeschlossen werden,
- bereits etablierte oder von Ausgangsspendern etablierte, begrenzt oder unbegrenzt wachsende Zelllinien,
- Blut von Vögeln und Säugern einschließlich des Menschen.

Das kompakte biologische Material wird mechanisch oder fermentativ in eine Suspension von Einzelzellen überführt, die für Monolayer- (stationär oder Roller-) kulturen oder Suspensionenskulturen genutzt werden, ebenso wie die bereits etablierten, begrenzt oder unbegrenzt wachsenden Zelllinien. Heparinisiertes Blut - insbesondere von akut antigenstimulierten Organismen - wird in Rollerkulturen verbracht.

Folgende Verfahren zur intrazellulären Erzeugung von BK sind verwendbar:

Selektion von Zellen auf BK-Bildung, protahierte Zellkultivierung, Einsatz in der Regel geringgradiger unphysiologischer Medien, Haltungsbedingungen, z.B. Temperatur, Zusatz physikalischer, chemischer oder biologischer Stimuli oder nichtzytopathogener, für den Zielorganismus nichtpathogener Viren.

Mit der Zellsedimentierung beginnt die Stofftrennung. In der ersten Stufe werden die Zellen von dem Medium getrennt. Das vor der Zellsedimentation benutzte Medium sollte weitgehend eiweißfrei oder -arm sein. Zur Lösung der Zellen von ihrer Haftung werden mechanische bzw. physikalische Methoden bevorzugt, letztlich wird das Zellsediment durch Zentrifugation gewonnen.

Die zweite Stufe der Stofftrennung, Trennung des BK von den übrigen Zellbestandteilen, beginnt mit der Zelldestruktion mittels Gefrier/Tau-Zyklen, Ultraschallbehandlung, der Zugabe von Detergentien oder mechanischen Methoden. Die Kontrolle der gelungenen vollständigen Freisetzung des BK aus Zellvakuolen geschieht mit dem Elektronenmikroskop im Negativkontrastverfahren. Anschließend erfolgt die Abtrennung von größeren Zelldetritus (Zellkern- und Zytoplasmabestandteile) durch Zentrifugation und Gewinnung des Überstandes, der den BK enthält.

Nach der Stofftrennung verblieben kleine bis mittelgroße Zellbestandteile (Reste von Mitochondrien, vom RER, von Zell- und Kernmembranen, Myelinfiguren u.a.) in Lösung. Eine fraktionierte Lösungsmittel- und Ultrazentrifugationsbehandlung schließt die Stofftrennung mit der Reindarstellung von BK im letzten Sediment nach Ultrazentrifugation ab. Die Resuspension von BK-Sediment erfolgt mit anorganischem Phosphatpuffer. Alternativ kann eine fraktionierte Fällung, z.B. mit PEG 6000, durchgeführt werden.

Die Prüfung auf Identität umfasst folgende Mindestanforderungen:
- Unter dem Elektronenmikroskop sind ausschließlich die Partikel des BK erkennbar;
- PAGE: charakteristische Muster;
- ELISA: quantifizierbare Reaktion gegen Antikörper mit typischem Kurvenverlauf,
- Prüfung auf Unschädlichkeit:
   Zellkultur: Fehlen zytotoxischer und zelltransformierter Anzeichen
- Tierversuch: Gesundes Überleben neugeborener Mäuse 4 Wochen post injektionen u.a.

Das Präparat wurde entsprechend den in den vorstehend genannten Schutzrechten angegebenen Verfahren hergestellt. Das Präparat wurde entsprechend der Erfordernisse der Arzneimittelherstellung in Glasampullen abgefüllt und bei Temperaturen unter 0 °C (für Langzeitlagerung bei ca. -80 °C) aufbewahrt. Je nach Anwendungsaufgabe kann BK-RiV durch an sich bekannte Verfahren in Protein- und RNA-Bestandteile getrennt und/oder durch thermische Behandlung die Destruktion der partikulären Grundstruktur von BK-RiV herbeigeführt werden, um durch komplette Freisetzung aller Inhaltsstoffe aus der partikulären Bindung, die biologischen Wirkungen auf den Organismus wesentlich zu verbessern bzw. erst zu ermöglichen entsprechend des Ersteinsatzes des Präparates.

Die BK-RiV-Präparate können nach an sich bekannten Verfahren, inaktiviert werden (z.B. thermisch im Wasserbad, chemisch mit Formalin) und eignen sich dann auch zur Behandlung von durch Tumore komplizierte AIDS-Erkrankungen

BK-RiV wird intramuskulär nach folgendem Schema angewendet:
Am ersten Tag: 1 Ampulle (= 1 ml),
am zweiten Tag: 2 Ampullen,
am dritten Tag: 2 Ampullen;
in einer Woche - Wiederholung,
dann jede Woche 2 Ampullen bis zur Normalisierung der weißen und roten Zellen im peripheren Blut; durch die wöchentliche bzw. 10-tägige, bzw. 14-tägige, bzw. 3-wöchentliche Kontrolle des Immunstatus des peripheren Blutes kann die Dosierung gesteuert werden und garantiert den stabilen Verlauf mit fallenden Applikationshäufigkeiten bis zur theoretisch möglichen Heilung. Die starke Zellproliferation erfordert die rechnerische Ermittlung und Realisierung der notwendigen ergänzenden Stoffzuführungen: Vitamine, Eisen, Spurenelemente (z.B. Selen u.a.) sowie die psychologische ärztliche Führung.

### Anwendungsbeispiel:

Patient A leidet seit Jahren an AIDS, bedingt durch eine HIV-Infektion, nach angeblich erfolgreich behandelter Krebskrankheit sanken - bei sehr schlechtem Allgemeinbefinden - die CD-4-T-Zellen auf 168, die CD-8-T-Zellen auf 505, die NK-Zellen auf 485, die Monozyten auf 334/µl ab, nach 29 Applikationen von BK-RiV innerhalb von 80 Tagen erhöhten sich diese Zellen auf ein stabiles Niveau bei sehr gutem Allgemeinbefinden (subjektiv: Arbeitsfähigkeit) - von CD-4-T-Zellen auf 328, von CD-8-Zellen auf 1585, von NK-Zellen auf 1065 und von Monozyten auf 686/µl, die Erythrozyten erhöhten sich um 1 Mill./µl. Es wurden keine unerwünschten Nebenwirkungen beobachtet.

## Patentansprüche

1. Verwendung von BK-RiV-Präparaten (Biokomplex - Reaktionsmuster in Vertebratenzellen) zur Herstellung eines Arzneimittels zur Therapie von AIDS, wobei die BK-RiV Präparate durch das folgende Verfahren
- Suche, Prüfung und Auswahl der Ausgangsspender für Zellkulturen, wie Embryonen, Feten, ausgenommen menschliche Embryonen und Feten oder Tiere bzw. deren Gewebe, Organe oder Tumore;
- Herstellung von begrenzt oder unbegrenzt wachsenden Zellkulturen;
- Stimulierung der Zellkulturen durch die Art und Weise der Kultivierung oder gezielter Einsatz biologischer, chemischer oder physikalischer Mittel;
- Gewinnung der Zellsedimente je nach Kultivierungsbedingungen unter weitgehender Zellerhaltung ohne Zusatz das Endprodukt belastender Stoffe;
- Zelldestruktion unter alleiniger Erhaltung von BK;
- Durchführung fraktionierter Zentrifugation, Ultrazentrifugation und Extraktion unter Sichtkontrolle mit dem Elektronenmikroskop im Negativkontrastverfahren;
- oder alternativ Durchführung von fraktionierten Fällungsverfahren und/oder anderer Stofftrennungsverfahren;
- Prüfung auf Identität und Unschädlichkeit erhältlich sind.

2. Verwendung eines Arzneimittels nach Anspruch 1, dadurch gekennzeichnet, dass die BK-RiV-Präparate in thermo- oder chemoinaktivierter Form eingesetzt werden.

3. Verwendung eines Arzneimittels nach Anspruch 1 und 2, dadurch gekennzeichnet, dass sie bei durch Tumore komplizierten AIDS-Erkrankungen eingesetzt werden.

## Claims

1. Use of BK-RiV- preparations (bio complex- reaction models in vertebral cells) for the production of a pharmaceutical to treat AIDS, so the BK-RiV- preparations can be obtained by the following procedure:
- search, check and selection of the initial donors for cell cultures as embryons, foeti with the exception of human embryons and foeti or animals or their tissue, organs or tumours;
- production of cell cultures growing in a limited or unlimited way;
- stimulation of cell cultures by the type of cultivation or planned use of biological, chemical or physical means;
- preparation of the cell sediments according to circumstances under which cultivation took place by a maximum of cell conservation without adding substances charging the final product;
- cell destruction by the sole conservation of the bio complex;
- realisation of a fractionated centrifugation, ultra-centrifugation and extraction under visual control by means of the electron microscope in the negative contrast procedure;
- or alternative realisation of fractionated precipitation procedure and/ or other substance separating procedure;
- testing for identity and harmlessness

2. Use of a pharmaceutical substance according to claim 1, characterised by the use of BK-RiV preparations in thermo- or chemoinactivated forms.

3. Use of a pharmaceutical substance according to claims 1 and 2, characterised by the use in case AIDS diseases complicated by tumours.

## Revendications

1. Emploi de préparations BK-RIV (mbcrcv - modèles biocomplexes de réaction dans des cellules vertébrales) dans le but de fabriquer un médicament pour le traitement du SIDA,
les préparations BK-RIV (mbcrcv) étant disponibles grâce à la procédure suivante :
- recherche, examen et choix de donneurs de départ pour les cultures de cellules tels qu'embryons, fétus, exceptés les embryons ou fétus humains et les animaux ou leurs tissus, organes ou tumeurs ;
- fabrication de cultures de cellules à croissance limitée ou non-limitée ;
- stimulation des cultures de cellules par la méthode de culture ou l'emploi précis de moyens biologiques, chimiques ou physiques ;
- production de sédiments cellulaires en fonction des conditions de culture et sous large préservation des cellules sans addition de substances menaçant la pureté du produit final ;
- destruction de cellules par la seule obtention de BK (biocomplexes) ;
- réalisation de centrifugation fractionnée, d'ultra-centrifugation et d'extraction avec contrôle optique à l'aidé du microscope électronique par le procédé de contraste en négatif ;
- ou, comme alternative, réalisation de procédures fractionnées de précipitation et/ou d'autres procédures de séparation des substances ;
- contrôle d'identité et de non-nocivité.

2. Emploi d'un médicament en fonction de la revendication 1, caractérisée par le fait que les préparations BK-RIV (mbcrcv) sont employées sous forme thermo- et chimioinactive.

3. Emploi d'un médicament en fonction des revendications 1 et 2, caractérisées par le fait qu'il est utilisé dans les cas de SIDA aggravés par des tumeurs.
